# EUROPEAN PATENT APPLICATION

(11) **EP 1 715 041 A1**
(43) Date of publication of application: **25.10.2006**
(21) Application number: 04711038.2
(22) Date of filing: 13.02.2004
(51) Int. Cl.: C12N 15/12

(54) **METHOD OF DETECTING CANCER CELL ACQUIRING DRUG-RESISTANCE**

(71) Applicant: BML, Inc., Tokyo 151-0051 (JP); Inazawa, Johji, Tokyo 178-0063 (JP)
(72) Inventor: INAZAWA, Johji, Tokyo 178-0063 (JP); YASUI, Kohichiro, Miyazu-shi, Kyoto 626-0033 (JP)
(74) Representative: Albrecht, Thomas
(86) International application number: PCT/JP2004/001574
(87) International publication number: WO 2005/078100

(57) **Abstract**

It is an object of the present invention to find out a novel gene marker by which a drug-resistant cancer cell can be detected and provide a means of efficiently and comprehensively detecting a drug-resistant cancer cell using this marker. In the present invention, gene amplifications or deletions have been analyzed in cancer cell strains resistant to drugs, which are anticancer drugs having particularly serious side effects and being administered to cancer patients at a high frequency (namely, camptothecins, cisplatins, etoposides, adriamycins (ADM), and cytosine arabinosides), and parent cancer cell strains. As a result, it was found out that the acquisition of drug-resistance to an anticancer drug in a test cancer cell can be detected by detecting amplification of one or more genes selected from ABC transporter genes and BCL2 family genes consisting of ABCA3 gene, ABCB6 gene, ABCB8 gene, ABCB10 gene, ABCC4 gene, ABCC9 gene, ABCD3 gene, ABCD4 gene, ABCE1 gene, ABCF2 gene, BCL2L2, BCL2L10, BCL2L1, and BCL2A1 which are novel gene markers relating to the acquisition of drug resistance of cancer cells.

SEQUENCE LISTING

<110> BML, INC.
Inazawa, Johji

<120> Method of Detecting Cancer Cell Acquiring Drug-Resistance

<130> PBM95PCT

<160> 4

<170> PatentIn version 3.2

<210> 1
<211> 20
<212> DNA
<213> Artificial

<220>
<223> Primer

<400> 1
tataagctga ggcagaaggg 20

<210> 2
<211> 20
<212> DNA
<213> Artificial

<220>
<223> Primer

<400> 2
tcagcactgt cctcactgat 20

<210> 3
<211> 21
<212> DNA
<213> Artificial

<220>
<223> Primer

<400> 3
agcctaccac ctcccctaga a 21

<210> 4
<211> 21
<212> DNA
<213> Artificial

<220>
<223> Primer

<400> 4
aagatcccct ccaccatccg a 21

## Description

### TECHNICAL FIELD

The present invention relates to a method of detecting cancer cells having acquired drug resistance to anticancer drugs, which have deletion or amplification of chromosomes.

### BACKGROUND ART

To date, a large number of anticancer drugs have been used to treat cancers. However, such drugs have been significantly problematic in terms of generation of serious side effects, the disappearance of drug effects or unresponsiveness due to the repetitive administration thereof, etc. In many cases, such problems are caused by the appearance of cells having acquired resistance to anticancer drugs. Drug resistance is caused by alteration in the genes of cancer cells. This is because such gene products decrease transportation of drugs into cells or promote discharge of drugs out of cells, promote inactivation or detoxication of drugs, suppress conversion of a prodrug (precursor) to an active-type drug, induce a decrease in a protein amount, to which a drug is targeted, or a decrease in the activity thereof, increase DNA repair activity, and suppress induction of apoptosis (Harrison, D. J.: Molecular mechanism of drug resistance in tumours, J. Pathol., 175, 7-12, 1995). There are also cases where multiple types of the aforementioned resistance acquisition take place simultaneously in cancer cells.

In drug-resistant cancer cells, the pumping of a drug from cells by a transporter localizing in the cell membrane, which is named as a "multidrug resistant protein (MDR and MRP)," plays an important role. For example, amplification of the ABCB1 (MDR1) or ABCC1 (MRP1) gene and the consequent overexpression thereof are recognized in a considerable number of drug-resistant cells (Kuwano, M., Uchiumi, T., Hayakawa, H., Ono, M., Wada, M., Izumi, H. and Kohno, K.: The basic and clinical implications of ABC transporters, Y-box-binding protein-1 (YB-1) and angiogenesis-related factors in human malignancies. Cancer Sci., 94, 9-14, 2003). A method of detecting the overexpression of a gene group encoding MDR using a DNA chip, a method of detecting a protein group using a specific antibody, and other methods, have been used as a method of detecting drug-resistant cancer cells. MDR belongs to a protein having ATP Binding Cassette (ABC) (which is referred to as an ABC protein). MDR discharges an amphipathic compound having a molecular weight of approximately 300 to 2,000 out of cells, using ATP hydrolysis energy.

On the other hand, when primary cancer cell strains and cancer cell strains are analyzed using Comparative genomic hybridization (CGH), an alteration in the specific region of chromosome associated with drug resistance is found (Wasenius, V. M., Jekunen, A., Monni, O., Joensuu, H., Aebi, S., Howell, S. B. and Knuutila, S.: Comparative genomic hybridization analysis of chromosomal changes occurring during development of acquired resistance to cisplatin in human ovarian carcinoma cells. Genes Chromosomes Cancer, 18, 286-291, 1997; Rao, P. H., Houldsworth, J., Palanisamy, N., Murty, V. V., Reuter, V. E., Motzer, R. J., Bosl, G. J. and Chaganti, R. S.: Chromosomal amplification is associated with cisplatin resistance of human male germ cell tumors. Cancer Res., 58, 4260-4263, 1998; Rooney, P. H., Stevenson, D. A., Marsh, S., Johnston, P. G., Haites, N. E., Cassidy, J. and McLeod, H. L.: Comparative genomic hybridization analysis of chromosomal alteration induced by the development of resistance to thymidylate syntase inhibitors. Cancer Res., 58, 5042-5045, 1998; Leyland-Jones, B., Kelland, L. R., Harrap, K. R. and Hioms, L. R.: Genomic imbalances associated with acquired resistance to platinum analogues. Am. J. Pathol., 155, 77-84, 1999; Struski, S., Doco-Fenzy, M., Trussardi, A., Masson, L., Gruson, N., Ulrich, E., Proult, M., Jardillier, J. C., Potron, G. and Comillet-Lefebvre, P.: Identification of chromosomal loci associated with non-P-glycoprotein-mediated multidrug resistance to topoisomerase II inhibitor in lung adenocarcinoma cell line by comparative genomic hybridization. Genes Chromosomes Cancer, 30, 136-142, 2001).

It is highly likely that amplification and deletion of genome, which are characteristic of drug-resistant cancer cells, contribute to resistance to anticancer drugs. Thus, it is extremely important to identify such a region. However, cancer cells exhibit various types of drug-responsiveness, and it is not sufficient to detect drug-resistant cells based on the currently obtained findings. Thus, there is no doubt that it is necessary to find out a novel marker. In addition, a method of systematically analyzing drug-resistant cancer cells, so as to efficiently and comprehensively examine the drug-resistant ability of cancer cells, has not yet been established.

Accordingly, it is an object of the present invention to find out a novel gene marker by which a drug-resistant cancer cell can be detected, and provide a means for efficiently and comprehensively detecting a drug-resistant cancer cell using this marker.

### DISCLOSURE OF THE INVENTION

The present inventors have analyzed gene amplifications or deletions in drug-resistant cancer cell strains which are resistant to anticancer drugs having serious side effects and being administered to cancer patients at a high frequency, namely, camptothecins (CPT), cisplatins (CDDP), etoposides (VP-16), adriamycins (ADM), and cytosine arabinosides (Ara-C), and parent cancer cell strains. As a result, the present inventors have discovered novel gene markers relating to the acquisition of drug resistance of cancer cells, thereby completing the present invention.

That is to say, the present invention provides a detection method of detecting the acquisition of the drug resistance of a test cancer cell to anticancer drugs, which comprises detecting amplification of one or more genes selected from ABC transporter genes and BCL2 family genes consisting of ABCA3 gene, ABCB6 gene, ABCB8 gene, ABCB10 gene, ABCC4 gene, ABCC9 gene, ABCD3 gene, ABCD4 gene, ABCE1 gene, ABCF2 gene, BCL2L2, BCL2L10, BCL2L1, and BCL2A1 in the test cancer cell (hereinafter referred to as "the present detection method" at times).

### (1) The present detection method

Among the above-listed anticancer drugs, the term "camptothecins" includes not only camptothecin, but also camptothecin derivatives such as DX8951 developed by Daiichi Pharmaceutical Co., Ltd., T-0128 developed by Menarini, Italy, and nogitecan hydrochloride developed by SmithKline Beecham, U.K. "Cisplatins" are also called platinum preparations (which contain platinum), and the term "cisplatins" includes cisplatin, vinblastine, and carboplatin. The term "etoposides" includes not only etoposide, but also an etoposide derivative, NK611 (etoposide derivative tablet) developed by Nippon Kayaku Co., Ltd. The term "cytosine arabinosides" includes not only cytosine arabinoside, but also the fluoride derivative thereof, gemcitabine, as a cytosine arabinoside derivative.

In the present detection method, among the above-listed genes defined as markers of acquired resistance to anticancer drugs, ABCA3 gene, ABCB6 gene, ABCB8 gene, ABCB10 gene, ABCC4 gene, ABCC9 gene, ABCD3 gene, ABCD4 gene, ABCE1 gene, and ABCF2 gene are all genes which encode ATP Binding Cassette (ABC) transporter proteins.

Proteins belonging to the ABC transporter family are involved in the transport of an extremely wide range of substances ranging from ions to high molecular weight proteins. It has been known that the ABCB1 (MDR1) gene and the ABCC1 (MRP1) gene increase the discharge of drugs from cells, so as to induce resistance to a large number of drugs (Ling, V.: Multidrug resistance: molecular mechanisms and clinical relevance, Cancer Chemother. Pharmacol., 40, S3-S8, 1997). In addition, it has also been known that the ABC11 gene is amplified in camptothecin-resistant leukemic cell strains, and that the overexpression thereof induces taxol resistance (Childs, S., Yeh, R. L., Hui, D. and Ling, V.: Taxol resistance mediated by transfection of the liver-specific sister gene of P-glycoprotein. Cancer Res., 58, 4160-4167, 1998).

In the present invention, it has been clarified that the aforementioned 10 types of ABC transporter genes (ABCA3 gene, ABCB6 gene, ABCB8 gene, ABCB10 gene, ABCC4 gene, ABCC9 gene, ABCD3 gene, ABCD4 gene, ABCE1 gene, and ABCF2 gene), in addition to the aforementioned 3 types of genes, are involved in the drug resistance. For example, in the chromosome 16p12-p13 region amplified in etoposide-resistant human colon cancer (HT-29/ETP) cells, ABCA3 gene is amplified and is overexpressed, rather than ABCC1 gene is. Accordingly, it has been clarified that ABCA3 gene is highly likely to be an amplicon (a region exhibiting gene amplification), rather than ABCC1 gene is. ABCC9 gene is a gene encoding the constitutional elements of ATP-sensitive K+ channels (Seino, S. ATP-sensitive potassium channels: a model of heteromultimeric potassium channel/receptor assemblies, Annu. Rev. Physiol., 61, 337-362, 1999). It has been clarified that ABCC9 gene is amplified in lung cancer (SK3/VP16) cells and ovarian cancer (SKOV3/VP) cells, which exhibit etoposide resistance. In the former cells, an increase in the expression of ABCC9 gene was confirmed. In addition, as described later, it has become clear that amplification of ABCA3 gene is an index of acquisition of drug resistance to etoposides, amplification of ABCB6 gene is an index of acquisition of drug resistance to camptothecins, amplification of ABCB8 gene is an index of acquisition of drug resistance to cisplatins, amplification of ABCB10 gene is an index of acquisition of drug resistance to camptothecins, amplification of ABCC4 gene is an index of acquisition of drug resistance to cisplatins, amplification of ABCD4 gene is an index of acquisition of drug resistance to adriamycins, amplification of ABCE1 gene is an index of acquisition of drug resistance to camptothecins, and amplification of ABCF2 gene is an index of acquisition of drug resistance to cisplatins.

Moreover, in the present detection method, the BCL2 family genes, which are the above-listed other types of genes defined as markers of acquired resistance to anticancer drugs, are genes which act to suppress apoptotic death due to anticancer drugs. Using such action, the BCL2 family genes impart multidrug resistance to cancer cells. Thus, it is assumed that BCL2 is a multidrug-resistant protein. It has been clarified that the overexpression of specific BCL2 family genes having anti-apoptotic action (BCL2 gene, BCLXL gene, and MCL1 gene) induces the resistance of cancer cells to clinically used drugs (Reed J. C. Dysregulation of apoptosis in cancer. J. Clin. Oncol., 17, 2941-2953, 1999).

In the present invention, it has been found that the aforementioned other types of BCL2 family genes (BCL2L2 gene, BCL2L10 gene, BCL2L1 gene, and BCL2A1 gene) can also be used as indexes of acquisition of drug resistance. That is to say, it has been clarified that amplification of BCL2L2 gene is an index of acquisition of drug resistance to camptothecins, cisplatins, etoposides, or cytosine arabinosides, amplification of BCL2L10 gene is an index of acquisition of drug resistance to camptothecins, cisplatins, or cytosine arabinosides, and amplification of BCL2L1 gene is an index of acquisition of drug resistance to camptothecins, cisplatins, etoposides, or cytosine arabinosides (as described later).

The above-listed ABC transporter genes and BCL2 family genes, which can be used as novel markers of acquisition of the drug resistance of cancer cells, are genes that are independent from one another. Taking into consideration a variety of mutations of intrinsic cancer cells, and as described in the examples of the present specification, every marker has a different type of acquisition of the drug resistance of cancer cells in many cases. Accordingly, it can be said that each of the above-listed gene markers is valuable as an independent gene marker. Conversely, a large number of such gene markers are combined with one another, and the obtained detection results are then considered. Thereby, it becomes also possible to comprehensively detect the resistance to anticancer drugs.

Furthermore, detection is carried out by combining the aforementioned newly provided gene markers of acquisition of resistance to anticancer drugs with the known gene markers, so that acquisition of the resistance of test cancer cells to anticancer drugs can be detected more precisely.

Examples of such known gene markers of acquisition of resistance to anticancer drugs may include: ABC transporter genes such as ABCB 1 gene, ABCC1 gene, or ABCB11 gene; BCL2 family genes such as BCL2 gene, MCL1 gene, or BCLXL gene; and DNA synthesis-associated genes such as DCK1 gene or TOP1 gene. Of these genes, the ABC transporter genes and the BCL2 family genes are as described above.

DNA synthesis-associated genes such as DCK1 gene or TOP1 gene are the following genes.

Cytosine arabinoside (Ara-C) is changed to an active phosphorylated form (1-β-D-arabinofuranosylcytosine 5'-triphosphate) by the action of deoxycytidine kinase (DCK). The active phosphorylated form is then incorporated into DNA, so as to exert a cell-killing effect. Accordingly, it has been reported that a decrease in the DCK enzyme activity induces resistance to Ara-C (Funato, T., Satou, J., Nishiyama, Y, Fujimaki, S., Miura, T., Kaku, M., and Sasaki, T: In vitro leukemia cell models of Ara-C resistance, Leuk. Res., 24, 535-541, 2000). The present specification also discloses that DCK gene is present in the form of a hemizygote in Ara-C-resistant leukemia (K562-AC) cells, and that as a result, the expression of DCK gene is decreased, inducing Ara-C resistance.

Topoisomerase (Topo) I and Topo IIα nick single-stranded DNA and double-stranded DNA, so as to separate the DNA strands during replication, thereby conducting DNA replication as scheduled. Camptothecin (CPT) inhibits Topo I, whereas etoposide (VP-16) inhibits Topo IIα, thereby inhibiting DNA replication. Based on the findings obtained so far, it has been known that the levels of intracellular Topo I and Topo IIα is in correlation with drug sensitivity. That is to say, it has been known that when the concentration of both enzymes is high, anticancer drug sensitivity is also high, and that when the concentration thereof is low, anticancer drug sensitivity is also low (Yanase, K., Sugimoto, Y., Tsukahara, S., Oh-Hara, T., Andoh, T., and Tsuruo, T.: Identification and characterization of a deletion mutant of DNA topoisomerase I mRNA in a camptothecin-resistant subline of human colon carcinoma, Jap. J. Cancer Res., 91, 551-559, 2000; McLeod, H. L., and Keith, W. N.: Variation in topoisomerase I gene copy number as a mechanism for intrinsic drug sensitivity., Br. J. Cancer, 74, 508-512, 1996; Withoff, S., Keith, W. N., Knol, A. J., Coutts, J. C., Hoare, S. F., Mulder, N. H., and de Vries, E. G.: Selection of a subpopulation with fewer DNA topoisomerase II a gene copies in a doxorubicin-resistant cell line panel., Br. J. Cancer, 74, 502-507, 1996; Sugimoto, Y., Tsukahara, S., Oh-hara T., Isoe, T., and Tsuruo, T: Decreased expression of DNA topoisomerase I in camptothecin-resistant tumor cell lines as determined by a monoclonal antibody, Cancer Res., 50, 6925-6930, 1990). As described later, the expression level of TOPI gene was low in 3 types of camptothecin-resistant cells, camptothecin-resistant colon cancer (HT-29/CPT) cells and camptothecin-resistant stomach cancer (St-4/CPT) cells, namely, in all the examined camptothecin-resistant cells. That is because the copy number of TOP1 genes was lower than that of the parent strains thereof. It has been known that amplification of the chromosome 20q region, in which the TOP1 gene localizes, takes place in colon cancer cells and stomach cancer cells (Knuutila, S., Bjorkqvist, A. M., Autio, K., Tarkkanen, M., Wolf, M., Monni, O., Szymanska, J., Larramendy, M. L., Tapper, J., Pere, H., El-Rafai, W., Hemmer, S., Wasenius, V. M., Vidgren, V., and Zhu, Y.: DNA copy number amplifications in human neoplasms: review of comparative genomic hybridization studies. Am. J. Pathol., 152, 1107-1123, 1998). In fact, the present specification also discloses that the copy number of the TOP 1 gene was significantly high in the sensitive cells of parent strains, that is, the copy number increased by 5 times in HT-29 cells and by 6 times in St-4 cells. Moreover, it was found that one pair out of two pairs of TOP1 genes was a TOP1 deletion mutant in camptothecin-resistant colon cancer (HT29/CPT) cells. As a result, it was revealed that a decrease in the expression of the TOP1 gene directly results in camptothecin resistance.

Moreover, in 5 types of etoposide-resistant cells, the expression of the TOP2A gene was lower than in the parent cell strains thereof, and the gene copy number on the chromosome was decreased to 3 copies. Accordingly, it is concluded that a decrease in the copy numbers of the TOP1 and TOP2A genes results in a decrease in the expression of these genes and brings on camptothecin resistance and etoposide resistance. Furthermore, in the case of the thymidine synthetase gene and dehydropyrimidine dehydrogenase gene as well, it is easily considered that a decrease in the copy numbers thereof induces drug resistance.

The present detection method can be carried out by the CGH method, the flow cytometry method, the ELISA method, the DNA chip method, or the quantitative PCR method, for example. Of these, the DNA chip method or the CGH method is preferable, and the CGH method is particularly preferable.

In the case of the "flow cytometry method," when ABC protein expressing in lymphatic leukemia cells is measured for example, lymphocytes are first collected by flow cytometry using an FITC (fluoresceinisothiocyanate)-labeled CD45 monoclonal antibody. Subsequently, using an anti-ABC protein antibody labeled with fluorescent dye such as Cy3, the ABC protein expressing in lymphatic leukemia cells can be measured. This method is suitable for detection of a protein expressing on the surface of a cancer cell membrane.

With regard to the "ELISA (enzyme-linked immunosorbent assay) method," the sandwich ELISA method using two types of anti-ABC protein antibodies is generally used, for example. Other than this method, the competitive method using a single type of anti-ABC protein may be used. The former method is a method, which comprises immobilizing one type of anti-ABC protein antibody on a plate, adding a cancer cell extract thereto, and detecting an ABC protein that binds to the above antibody using the other type of antibody. Specifically, it is a method, which involves labeling the other antibody or detecting the antibody using an alkaline phosphatase-labeled antibody, a peroxidase-labeled antibody, or the like. Since a specific antibody is used in this method, this method can be applied to any types of proteins expressing in cells.

The "DNA chip method" is a method of quantifying mRNA expressing in cancer cells. For example, a synthetic oligonucleotide having a part of a gene encoding the ABC protein group is immobilized on a substrate (cDNA can also be immobilized thereon). When cDNA is synthesized from RNA prepared from cancer cells using reverse transcriptase, labeling is carried out. The thus labeled cDNA is allowed to hybridize with the synthetic oligonucleotide immobilized on the substrate, and the amount of the bound label is then scanned, thereby measuring the expression level of mRNA. The present invention also provides a DNA chip that can be used in this DNA chip method, namely, a DNA-fixed substrate on which "DNA (which may be either cDNA or a synthetic oligonucleotide), which comprises one or more types of genes selected from among ABC transporter genes and BCL2 family genes, which consist of ABCA3 gene, ABCB6 gene, ABCB8 gene, ABCB10 gene, ABCC4 gene, ABCC9 gene, ABCD3 gene, ABCD4 gene, ABCE1 gene, ABCF2 gene, BCL2L2 gene, BCL2L10 gene, BCL2L1 gene, and BCL2A1 gene" has been fixed. (The material of the substrate, the production process, and the like, are the same as those of a DNA-fixed substrate used as a CGH array, which will be described later.)

The "quantitative PCR method" is a method, which comprises amplifying a region comprising a specific gene of test DNA (a gene which is amplified or deleted as a result of acquisition of the drug resistance of cancer cells) by the real-time PCR method, quantifying the real-time PCR amplified product derived from the above test DNA and a control product (a gene amplified product which is derived from the DNA of normal cells, and is obtained as a result of gene amplification by the PCR method using the same gene amplification primers under the same conditions), and comparing them, so as to detect amplification or deletion of the above specific gene.

### (2) The present detection method, using CGH (Comparative Genomic Hybridization) method or DNA chip method

The present detection method in this embodiment is a method of detecting cancer cells having acquired drug resistance, which comprises: allowing control DNAs and the DNA of a test cancer cell used as a target of detection of acquisition of drug resistance, each of which was labeled with each different fluorescent dye, to simultaneously contact with the aforementioned DNA-fixed substrate, so as to conduct hybridization; and quantitatively detecting amplification or deletion of a specific region of the test DNA by using the fluorescent dye obtained as a result of the hybridization as an index.

### Human DNA-fixed substrate used in CGH method or DNA chip method

Human DNA to be fixed on a DNA-fixing substrate (the present invention also provide such a DNA-fixed substrate) used in such DNA detection methods can be selected depending on the type of a detection method selected. That is to say, when the DNA chip method is applied in one embodiment of the present detection method, cDNA or a synthetic oligonucleotide is used. On the other hand, when the CGH method is applied, genomic DNA is used.

It is possible to conduct collection and preparation of DNA according to common methods. In particular, in the case of control DNA, a commercially available product can also be used.

A particularly preferred embodiment of the present detection method includes application of the CGH method using a substrate, on which each of multiple types of gene amplified products obtained from BAC (Bacterial Artificial Chromosome) DNA, YAC (Yeast Artificial Chromosome) DNA, or PAC (Phage Artificial Chromosome) DNA, which has a specific genomic DNA region, has been fixed.

That is to say, as cancer cells acquire resistance to anticancer drugs, an amplified or deleted genomic region becomes enormous in some cases. Thus, it is necessary that genomic DNA reflecting an enormous gene as a single unit be fixed on the present fixing substrate. It is also necessary to use BAC DNA, YAC DNA, or PAC DNA (hereinafter referred to as "BAC DNA etc." at times) having an allowable gene incorporation volume, which can satisfy the above necessity. BAC DNA etc. which can be used in the present invention may be selected from a gene library, which is obtained by incorporation of genome to be used according to a common method, or it may also be selected from a commercially available gene library. BAC DNA or PAC DNA, which should be selected depending on purpose, will be described later. A host into which a selected clone has been incorporated, is cultured, and BAC DNA etc. can be then purified.

The amount of the thus commonly obtained BAC DNA etc. is too small to produce a large number of genomic DNA-fixed substrates and to commercialize it. Thus, it is necessary to obtain the above DNA as a gene amplified product (such a gene amplification process is also referred to as a "process of inexhaustibly producing gene amplified products"). In such a process of inexhaustibly producing gene amplified products, first, BAC DNA etc. is digested with 4 nucleotides recognition enzyme such as RsaI, DpnI, or HaeIII. Thereafter, an adapter is added thereto, so as to carry out ligation. The adapter is an oligonucleotide consisting of 10 to 30 nucleotides, and preferably consisting of 15 to 25 nucleotides. It is necessary that a double strand have a complementary sequence, and that a 3'-terminal oligonucleotide on the blunt end formation side be phosphorylated after annealing. Subsequently, using a primer having the same sequence portion as that of either one oligonucleotide of the adaptor, the above DNA is amplified by the PCR (Polymerase Chain Reaction) method, so as to produce gene amplified products inexhaustibly. On the other hand, an aminated oligonucleotide consisting of 50 to 70 nucleotides, which is characteristic of BAC DNA etc., can be used as a detection probe.

The thus inexhaustibly produced BAC DNA etc. (genomic DNA, cDNA, and a synthetic oligonucleotide in embodiments other than in the above embodiment is also applicable) is fixed on a substrate, and preferably on a solid substrate, so as to produce a desired DNA-fixed substrate.

Examples of a solid substrate may include a glass, a plastic, a membrane, and a three-dimensional array. A glass substrate such as a slide glass is preferable. It is more preferred that a solid substrate such as a glass is coated by agglutination of poly-L-lysine, aminosilane, gold, aluminum, etc.

The concentration of the aforementioned inexhaustible DNA (genomic DNA, cDNA, and a synthetic oligonucleotide in embodiments other than in the above embodiment is also applicable) spotted on a substrate is preferably between 10 pg/µl and 5 pg/µl, and more preferably between 1 ng/µl and 200 ng/µl. The amount of the above DNA spotted thereon is preferably between 1 nl and 1 µl, and more preferably between 10 nl and 100 nl. The size and form of each spot fixed on the substrate are not particularly limited. For example, the size of each spot may be between 0.01 and 1 mm in diameter, and the form of each spot seen from above may be a round or oval form. The thickness of a dry spot is not particularly limited, and it is between 1 and 100 µm. The number of spots is not particularly limited, and it is between 10 and 50,000, and more preferably between 100 and 5,000, per substrate used. Each type of DNA is spotted in a range from singular to quadruplicate. It is preferable to spot in a duplicate or triplicate manner.

Dry spots can be prepared, for example, by dropping the inexhaustibly produced BAC DNA etc. (genomic DNA, cDNA, and a synthetic oligonucleotide in embodiments other than in the above embodiment is also applicable) onto a substrate using a spotter, so as to form a plurality of spots, and then drying the spots. Examples of a spotter used herein may include an ink-jet printer, a pin-array printer, and a bubble-jet printer. Of these, an ink-jet printer is preferably used. For example, high throughput ink-jet dispensing system SQ series of Cartesian Technologies (U.S.A.) can be used.

As described above, the inexhaustibly produced BAC DNA etc. (genomic DNA, cDNA, and a synthetic oligonucleotide in embodiments other than in the above embodiment is also applicable) can be fixed on a substrate, and preferably on a solid substrate, so as to produce a desired DNA-fixed substrate.

### Detection embodiment

When amplification or deletion of a specific region of test DNA is quantitatively detected using the aforementioned DNA-fixed substrate, there is used a detection method, which comprises: allowing control genomic DNAs (genomic DNAs or the like of normal cells or normal tissues) and the test genomic DNAs (genomic DNA or the like of a cancer cell or cancer tissue used as a target of detection of acquisition of drug resistance), each of which was labeled with each different fluorescent dye, to simultaneously contact with the present fixed substrate on which fragments of genomic DNA or the like of a specific region have preferably comprehensively been fixed, so as to conduct hybridization; and quantitatively detecting amplification or deletion of a specific region of the test genomic DNA or the like by using the fluorescent dye obtained as a result of the hybridization as an index.

By quantitatively or qualitatively detecting amplification or deletion of the specific region of the test genomic DNA or the like, the presence or absence of acquisition of the resistance of the test cancer cell to anticancer drugs, the type of such resistance, and the like, can be clarified.

A commercially available product can also be used as human genomic DNA or the like. Such human genomic DNA can also be extracted from the plasma collected from a healthy subject according to a common method. Further, it is also possible to obtain such human genomic DNA from the normal tissues of a cancer patient according to a common method.

Control genomic DNA or the like (the genomic DNA of normal cells or tissues, or the like) and test genomic DNA or the like (the genomic DNA of cancer cells or tissues used as targets in which acquisition of drug resistance is detected, or the like) can be labeled with different fluorescent dyes, such as Cy3 and Cy5, according to a common method (the nick-translation method using dCTP, for example). A labeling kit used for such a nick-translation method using dCTP is commercially available from Pan Vera (Japanese distributor: Takara Shuzo Co., Ltd.), Invitrogen (CA, U.S.A.), or the like. When the labeled DNA is hybridized with DNA printed on a CGH array, it is more preferable to add Cot-1 DNA, formamide, dextran sulfate, SSC (150 mM NaCl/15 mM sodium citrate), Yeast t-RNA, and SDS (sodium deoxysulfate). In addition, it is preferable to denature a solution containing the labeled DNA by heating, followed by addition of the resultant. It is possible to place a container for hybridization on a platform equipped with locking function. It is more preferable to use a container capable of evenly contacting with a small amount of solution on the present fixed substrate, for example, to use "Hybriman." The temperature applied during hybridization is preferably between 30°C and 70°C, and more preferably between 38°C and 45°C. The time required for hybridization is preferably between 12 and 200 hours, and more preferably between 40 and 80 hours. The present fixed substrate can be washed with formamide, an SSC solution, or the like, at a room temperature. Such washing of the present fixed substrate is an important step of reducing non-specific signals to the minimum. It is more preferable that the fixed substrate be washed at room temperature and be then washed with the same washing solution at a temperature between 40°C and 60°C, that it be further washed with a solution containing SSC-SDS at 50°C, that the resultant be left at rest in a solution containing a phosphate buffer/NP-40, and that the resultant be finally stirred in a solution containing SSC.

Moreover, in the present detection method, in order to grasp the contents of generation of fluorescence on the present fixed substrate after hybridization, a scanner can be used, for example. An example of such a scanner is GenePix 4000B (Amersham Biosciences K.K., Tokyo). In order to analyze the results, it is preferable that when the control genomic DNA is labeled with Cy5 and the genomic DNA derived from test cancer cells is labeled with Cy3, the mean value of Cy3 fluorescence intensity on the present fixed substrate and the mean value of Cy5 fluorescence intensity thereon be corrected to the same value. Furthermore, it is preferable that the ratio of Cy3/Cy5 be obtained on each BAC DNA (each pixel), and that it be expressed as a Log₂ratio value. Using the intensity ratio of the above fluorescence (fluorescent dye by the naked eye) as an index, it becomes possible to quantitatively (of course, qualitatively also) detect whether or not amplification or deletion of a specific portion of a gene as a detection target is observed in a genomic DNA sample obtained from test cancer cells.

### BRIEF DESCRIPTION OF THE DRAWINGS

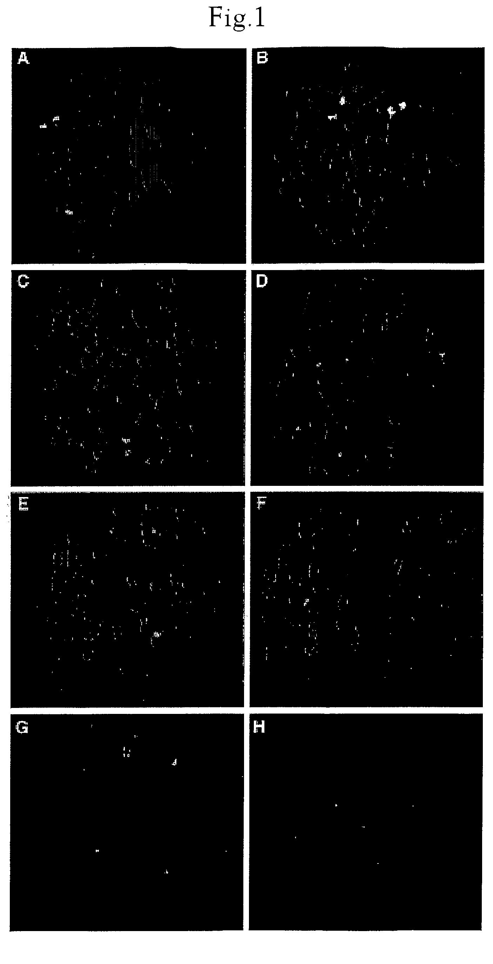

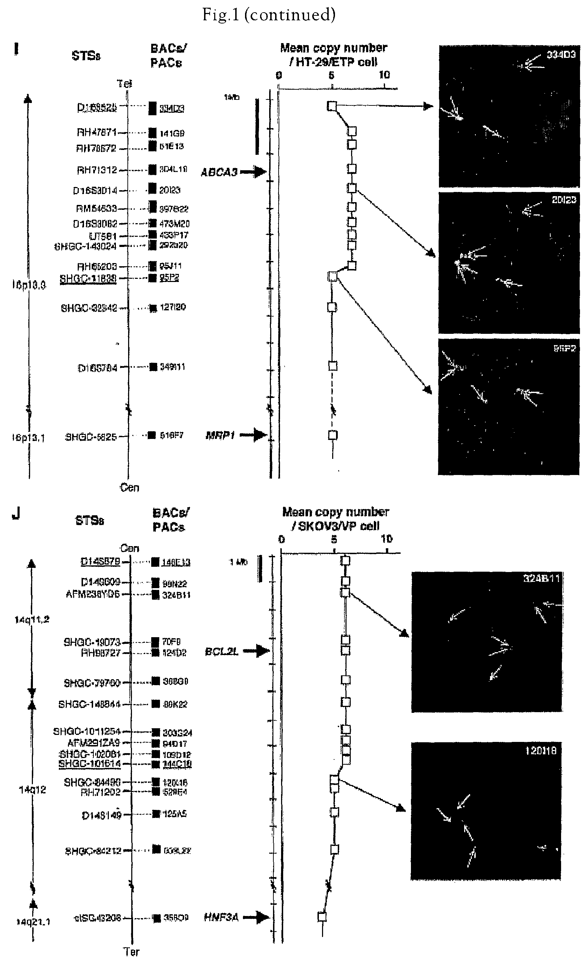

Figure 1 is a group of photographs showing the results obtained by analyzing gene amplification and deletion by fluorescence *in situ* hybridization using the metaphase chromosomes of the following cell lines:
   (A) human colon cancer cell HT-29 line; (B) etoposide-resistant human colon cancer cell HT 29/ETP line; (C) human ovarian cancer cell SKOV3 line; (D) etoposide-resistant human ovarian cancer cell SKOV3/VP line; (E) human leukemia K562 line; (F) cytosine arabinoside-resistant human leukemia K562/AC line; (G) human colon cancer cell HT-29 line; and (H) camptothecin-resistant human colon cancer cell HT-29/CPT line.

In the HT-29 cells, green fluorescence derived from the ABCA3 gene and red fluorescence derived from the ABCC1 (MRP1) gene were detected at 3 sites (A). In the HT-29/ETP cells, green fluorescence derived from the ABCA3 gene was detected at 7 sites in all the chromosomes, and red fluorescence derived from the ABCC1 (MRP1) gene was detected at 5 sites in all the chromosomes (B). In the SKOV3 cells, green fluorescence derived from the ABCA3 gene was detected at 7 sites, and red fluorescence derived from the HNF3A gene was detected at 4 sites (C). In the SKOV3/VP cells, green fluorescence derived from the BCL2L2 gene was detected at 6 sites, and red fluorescence derived from the HNF3A gene was detected at 4 sites (D). In the K562 cells, green fluorescence derived from the DCK gene was detected at 1 site (E). In the K562/AC cells, green fluorescence derived from the DCK gene was detected at 1 site (F). In the HT-29 cells, green fluorescence derived from TOP1 was detected at 5 sites (G). In the HT-29/CPT cells, green fluorescence derived from TOP1 was detected at 2 sites (H).

In photographs (A) and (B), since the ABCA3 gene and the ABCC1 gene localize extremely closely to each other, the green fluorescence and the red fluorescence overlap, so as to present a yellow color. Bacterial artificial chromosomes (BAC) and P1-artificial chromosomes (PAC), which were used in fluorescence *in situ* hybridization, are shown below.

| Gene name | BAC/PAC name |
|---|---|
| ABCA3 gene (ABC family gene) | RP11-304L19 |
| ABCC gene (ABC family gene) | RP11-516F7 |
| HNF3A gene (Hepatocyte nuclear factor 3 family gene) | RP11-356O9 |
| BCL2L2 gene (BCL2 family gene) | RP11-124D2 |
| DCK gene (deoxycytidine kinase gene) | RP11-499N1 |
| TOP 1 gene (topoisomerase gene) | RP1-1J6 |

An amplicon (a region exhibiting gene amplification) on the HT-29/ETP cell chromosome is mapped on 16p12-p13, and the ABCA3 gene is adjacent to the ABCC1 (MRP1) gene (I). An amplicon on the SKOV3/VP cell chromosome is mapped on 14q11-q21, and the BCL2L gene and the HNF3A gene exist (J).

Fluorescence *in situ* hybridization (FISH) was carried out according to the method described in Yasui, K., Imoto, I., Fukuda, Y., Pimkahaokham, A., Yang, Z. Q., Naruto, T., Shimada, Y, Nakamura, Y, and Inazawa: Identification of target genes within an amplicon at 14q12-q13 in esophageal squamous cell carcinoma. Genes Chromosomes Cancer, 32, 112-118, 2001.

In Figure 1(I) and 1(J), BAC and PAC (whose numbers are shown in the figures) were subjected to FISH using labeled probes, and detection sites of etoposide-resistant colon cancer (HT-29/ETP) cells and etoposide-resistant ovarian cancer (SKOV3/VP) cells on the chromosomes are expressed with the mark → in the figures. Regarding the results, amplification of the present chromosomal region in the HT-29/ETP cells was expressed as a mean copy number, using localizing sites on the chromosomes, STS (Sequence-tagged site) existing in the regions, the BAC and PAC clone names covering the regions, and such BAC and PAC used as probes. The chromosomal regions in which the ABCA3 gene, the MRP1 gene, the BCL2L gene, and the HNF3A gene exist are shown with arrows. The BAC name or PAC name used as a probe is described on the upper right hand side of each of the fluorescence photographs of the HT-29/ETP cells and the SKOV3/VP cells.

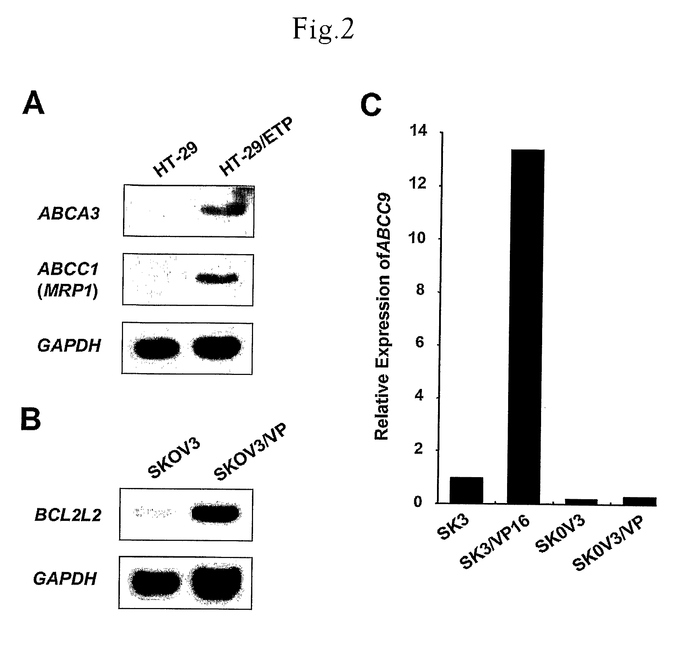

Figure 2 is a view showing overexpression of the ABCA3 gene, the ABCC1 (MRP1) gene, the BCL2L2 gene, and the ABCC9 gene, in drug-resistant cell lines.

Figure 2(A) shows the analysis of expression of the ABCA3 and ABCC1 (MRP) genes in the HT-29 cells and HT-29/ETP cells by the Northern blot method. 2 µg of poly (A) RNA was prepared from such 2 types of cells, and the Northern blot was carried out according to a common method, so as to measure the expression level of the aforementioned 2 types of genes. As probes for detecting the ABCA3 gene and the ABCC1 (MRP1) gene, IMAGE clones 179576 and 2205297 (Incyte Genomics, Palo Alto, CA) were used, respectively. GAPDH (glyceraldehyde-3-phosphate dehydrogenase) gene probe was used as a standard for correcting errors among samples.

Figure 2(B) shows the analysis of expression of the BCL2L2 gene in the SKOV3 cells and SKOV3/VP cells by the Northern blot method. 2 µg of poly (A) RNA was prepared from such 2 types of cells, and the Northern blot was carried out according to a common method, so as to measure the expression level of the BCL2L2 gene. Probes used for detecting the BCL2L2 gene were prepared by amplification by PCR (polymerase chain reaction) using two types of primers (forward: 5'-TATAAGCTGAGGCAGAAGGG-3' (SEQ ID NO: 1); and (reverse: 5'-TCAGCACTGTCCTCACTGAT-3' (SEQ ID NO: 2)). A GAPDH gene probe was used as a standard for correcting errors among samples.

Figure 2(C) shows the analysis of expression of ABCC9 mRNA using the SK3 cells, SK3/VP cells, and SKOV3/VP cells. RNA was prepared from these cells, and the ABCC9 gene mRNA was quantified by the real-time reverse transcription PCR method (Yasui, K., Arii, S., Zhao, C., Imoto, I., Ueda, M., Nagai, H., Emi., M. and Inazawa J.: TFDP1, CUL4A and CDC16 identified as targets for amplification at 13q34 in hepatocellular carcinomas, Hepatology, 35, 1476-1484, 2002). In order to standardize differences among samples, the ratio between the expression level of the ABCC9 gene and that of the control gene (GAPDH) was obtained. Thereafter, the ratio (ABCC9 expression level/GAPDH expression level) in the SK3 cells was defined as 1, and the relative ratio of ABCC9 gene expression level in each type of cells is shown in the figure.

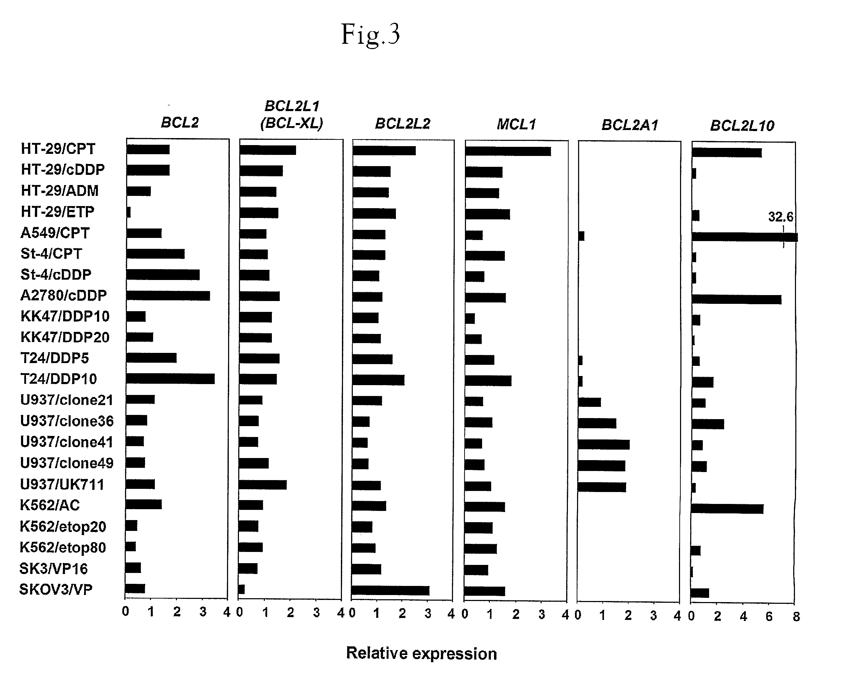

Figure 3 is a view showing expression of the BCL2 family genes in drug-resistant cell lines, which are compared with that in the parent lines thereof.

The ratio between the expression level of the control gene (GAPDH) and that of a gene of interest in each type of cells was obtained. The ratio of the parent line of each drug-resistant cell line was defined as 1, and the ratio of the expression level of each type of drug-resistant cells was then indicated. The gene expression level was measured by real-time reverse transcription PCR.

In the left column, drug-resistant cancer cell names are described. In the upper column of each bar graph, gene names to be reviewed are described. In the lower column, the expression level of each gene described in the upper column is described as a value compared with that of a parent line.

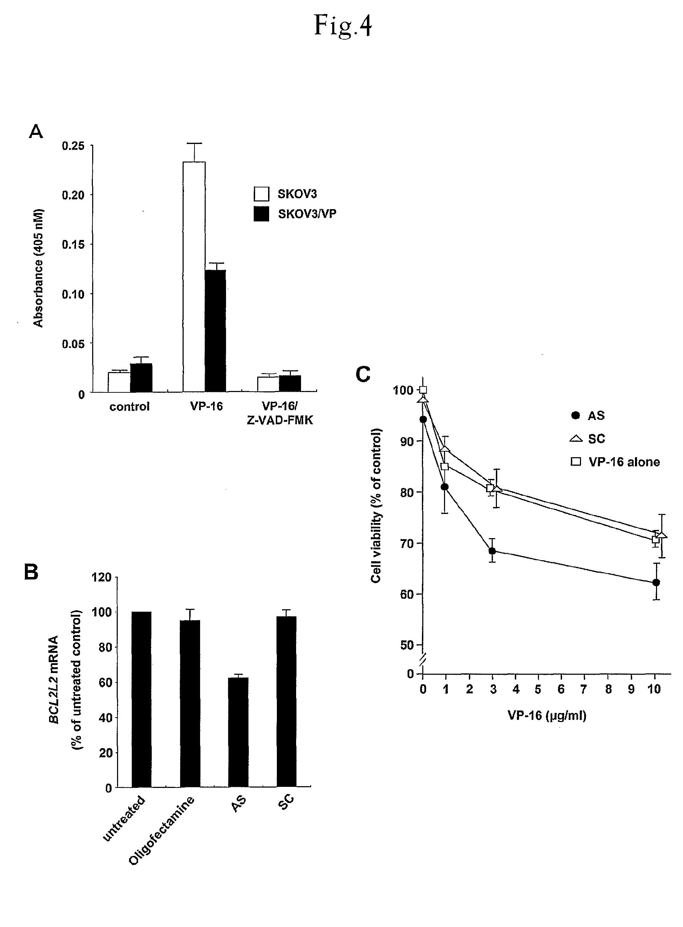

Figure 4 is a view showing caspase activity and the relationship between BCL2 family gene expression and etoposide resistance.

Figure 4(A) shows the caspase activity of ovarian cancer cells (SKOV3 and SKOV3/VP) treated with etoposide (VP-16).

Caspase activity was measured using a calorimetric CaspACE assay system (Promega Co., Madison, WI, U.S.A.) in accordance with the instructions included therewith. SKOV3 cells and SKOV3/VP cells were cultured for 24 hours in the presence of 100 µg/ml VP-16, or VP-16 and the caspase inhibitor Z-VAD-FMK, or in the presence of only a medium (control). The cells were dissolved in a lysis buffer, and the resultant was then subjected to centrifugation at 4°C at 15,000 x g for 20 minutes. The supernatant (containing 100 µg of protein) was incubated with a substrate (Ac-DEVD-pNA) used for the measurement of caspase activity at 37°C for 4 hours. Thereafter, the absorbance at 405 nm was measured, so as to obtain the activity.

Figure 4(B) shows the expression level of BCL2L2 mRNA in the SKOV3/VP cells treated with BCL2L2 antisense oligonucleotide.

The SKOV3/VP cells were transfected by each of the following treatments: untreatment; a treatment with oligofectamine which is a transfection reagent; transfection with a BCL2L2 antisense oligonucleotide having a phosphorothioate skeleton (a sequence portion from positions 2374 to 2394 of BCL2L2 cDNA: 5'-AGCCTACCACCTCCCCTAGAA-3' (SEQ ID NO: 3)) by a treatment with oligofectamine; and transfection with a control oligonucleotide having a phosphorothioate skeleton with a scramble sequence (5'-AAGATCCCCTCCACCATCCGA-3' (SEQ ID NO: 4)) by a treatment with oligofectamine. In each case, RNA was extracted, and the amount of BCL2L2 mRNA was measured by the real-time reverse transcription PCR method.

Figure 4(C) shows an increase in the sensitivity of the SKOV3/VP3 cells to etoposide VP-16 due to suppression of the expression of BCL2L2 gene.

The SKOV3/VP3 cells were transfected with a BCL2L2 antisense oligonucleotide having a phosphorothioate skeleton by an oligofectamine treatment. The same above cells were transfected with a control oligonucleotide having a phosphorothioate skeleton with a scramble sequence by an oligofectamine treatment. Also, the same above cells were treated under no treatment conditions. 24 hours later, the cells were cultured for 48 hours in the presence of VP-16 in each concentration as shown in the figure. 72 hours after the transfection, the survival rate of the SKOV3/VP3 cells was measured by the tetrazolium assay method using Cell-Counting Kit-8 (Dojindo Laboratories, Kumamoto). The survival rate was indicated with %. Such % was obtained by dividing the absorbance measured under the aforementioned treatment conditions by the absorbance measured under conditions consisting of no addition of VP-16 and untreatment. Each value is the mean value of 5 times of experiments, and the value is indicated with the mean value on each plot ± SD.

### BEST MODE FOR CARRYING OUT THE INVENTION

### [Example 1] Alternation in specific genomic DNA region which is specifically recognized in drug-resistant cancer cells

The subtractive CGH method is a method, which comprises comparing the copy number of parent cell line genome with the copy number of each genomic regions of the drug-resistant cell induced from the parent cell line, followed by subtraction, so as to quantitatively calculate the degree of gene amplification and deletion. Using this method, alterations in gene amplification or deletion, which were found in 23 types of drug-resistant cancer cells when compared with the parent lines, are shown in Table 1.

**(Table 1) Regions comprising chromosomal abnormality detected by applying subtractive CGH method to the parent line cancer cell genome and drug-resistant cancer cell genome induced from the parent line cancer cells**

| No. | Cell line | Drug name | Amplified region | Deleted region |
|---|---|---|---|---|
| 1 | HT-29/CPT | CPT | 1q, 4q24-35 | 11p, 11q23-25, 20p, 20q |
| 2 | HT-29/cDDP | CDDP | 1q31-32, 1q42-44, 3p21-26, 5p14-15 | 11p14-15, 15q11-21 |
| 3 | HT-29/ADM | ADM | 6q16-22, 11p, 13q21-22, 14q21-32, 18q12-21 | 7p21-22, 8q23-24, 9q34 |
| 4 | HT-29/ETP | VP-16 | 16p12-13 | None |
| 5 | A549/CPT | CPT | 2q21-37 | 9q31-34, 11p11-14, 11q14-22, 16q, Xp, Xq |
| 6 | St-4/CPT | CPT | None | 11p |
| 7 | St-4/cDDP | CDDP | None | None |
| 8 | A2780/cDDP | CDDP | None | Xp, Xq |
| 9 | KK47/DDP10 | CDDP | Sq14-35, 6q24-27, 13q22-34, 14q22-32 | 5p |
| 10 | KK47/DDP20 | CDDP | 6q21-27, 13q22-34 | 5p |
| 11 | T24/DDP5 | CDDP | 4p15-16 | 13q |
| 12 | T24/DDP7 | CDDP | 4p15-16 | None |
| 13 | T24/DDP10 | CDDP | 3p22-26, 3q13-24, 4p, 5q14-15, 7q34-36, 12q23-24 | 13q14-34, 15q11-21 |
| 14 | U937/clone21 | VP-16 | 2p21-25 | None |
| 15 | U937/clone26 | VP-16 | None | None |
| 16 | U937/clone41 | VP-16 | None | None |
| 17 | U937/clone49 | VP-16 | None | 18q21 |
| 18 | U937/UK711 | VP-16 | None | None |
| 19 | K562/AC | Ara-C | 1p21-31, 7q, 11p, 15q14-24, 20p13, Xp, Xq | 2q, 4p, 4q, 5p, 11q13-23 |
| 20 | K562/etop20 | VP-16 | 16p | None |
| 21 | K562/etop80 | VP-16 | 14q21-32, 16p, 16q12-13 | 9p21-24, 12q21-24 |
| 22 | SK3/VP16 | VP-16 | 2q36-37, 4q21-35, 7q21-31 | 3p, 5p, 10q, 20q |
| 23 | SKOV/VP | VP-16 | 1p13-22, 5q31-35, 11q23-25, 12p,14q11-21,17p | 20q12-13 |

| | | | | |
|---|---|---|---|---|
| (Abbreviated symbols) CPT: camptothecin; CDDP: cisplatin; ADM: adriamycin; VP-16: etoposide; Ara-C: cytosine arabinoside; HT-29: colon cancer cells; A549: lung cancer cells; SK3: lung cancer cells; St-4: stomach cancer cells; A2780: ovarian cancer cells; SKOV3: ovarian cancer cells; KK47: bladder cancer cells; T24: bladder cancer cells; K562: leukemia cells; and U937: leukemia cells | | | | |

In 19 types of drug-resistant cells (which correspond to 83% of all types of cells), the DNA copy number was changed. The number of sites, at which abnormality took place, was between 0 and 12 sites in all chromosomes of a single cell, and the mean value was 3.7 sites. Gene amplification took place at 0 to 7 sites, and the mean value was 2.1 sites. Deletion took place at 0 to 6 sites, and the mean value was 1.6 sites. In 4 types of cells (which correspond to 17% of all types of cells), gene amplification was found in chromosome 14q, and in 3 types of cells (which correspond to 13% of all types of cells), such gene amplification was found in chromosomes 4p, 6q, 7q, and 13q. Amplification of several or more copies was detected in the chromosome 2q23-q34 region of camptothecin-resistant lung cancer (A549/CPT) cells, in the chromosome 4p region of cisplatin-resistant bladder cancer (T24/DDP10) cells, in the chromosome 7q21-q22 region of etoposide-resistant lung cancer (SK3/VP16) cells, and in the chromosome 15q21-q24 region of cytosine arabinoside-resistant leukemia (K562/AC) cells. Gene deletion was detected in the chromosomes 5p and 11p of 4 types of cells (which correspond to 17% of all types of cells), and it was detected in the chromosomes 11q and 20q of 3 types of cells (which correspond to 13% of all types of cells). 8 types of cisplatin-resistant cells were examined, and the minimum common regions of chromosome amplification were 3p22-p26 (common to colon cancer HT-29/cDDP cells and bladder cancer T24/DDP10 cells), 4p15-p16 (common to bladder cancer T24/DDP5, T24/DDP7, and T24/DDP19 cells), and 5p15 (common to colon cancer HT-29/cDDP cells and bladder cancer T24/DDP10 cells). The chromosomal regions of deletion, which were common to cisplatin-resistant cells, were 5p (common to bladder cancer KK47/DDP10 cells and KK47/DDP20 cells), 13q14-q34 (common to bladder cancer T24/DDP5 and T24/DDP10 cells), and 15q11-q21 (common to bladder cancer HT-29/CDDP and bladder cancer T24/DDP10 cells). Moreover, 2 types of alteration of chromosomal regions were detected in 10 types of etoposide-resistant cancer cells. Such alternations were amplification of the chromosomal 16p12-p13 region (common to colon cancer HT-29/ETP cells, leukemia K562/etop20 cells, and leukemia K562etop80 cells) and deletion of the chromosomal 20q12-q13 region (common to lung cancer SK3/VP cells and ovarian cancer SKOV3/VP cells). Deletion of the chromosomal 11p11-p14 region was observed in all 3 types of camptothecin-resistant cells (common to colon cancer HT-29/CPT, lung cancer A549/CPT, and stomach cancer St-4/CPT cells). Subsequently, a gene group, which is closely associated with drug resistance comprised in such chromosomal regions comprising amplification and deletion, was identified.

### [Example 2] Amplification of ABC transporter genes and overexpression thereof

To date, 48 types of human genes have been known as human genes encoding the ABC transporter (Dean, M., Hamon, Y. and Chimini, G.: The human ATP-binding cassette (ABC) transporter superfamily, J. Lipid Res., 42, 1007-1017, 2001). In the drug-resistant cells examined herein, 20 types of ABC transporter genes were amplified. Among such 20 types of ABC transporter genes, amplification of 17 types of genes was confirmed by the FISH method.

13 types of ABC transporter genes were reliably amplified in 19 types of drug-resistant cells, when compared with parent lines (Table 2).

**(Table 2) Cell line list showing the results that when parent line cancer cells are compared with drug-resistant cancer cells induced from such parent line cancer cells, ABC transporter genes and Bcl-2 family genes are amplified in the latter cells.**

| Family | Gene name | Alias name | Localized chromosomal region | BAC/PAC name | Cell line | DNA copy number* |
|---|---|---|---|---|---|---|
| ABC transporter | | | | | | |
| | ABCA3 | | 16p13.3 | RP11-304L19 | HT29/ETP | 7 (3) |
| | | | | | K562/etop80 | 5 (4) |
| | ABCB1 | MDR1 | 7q21 | RP11-212B1 | SK3/VP16 | 11 (3) |
| | ABCB6 | | 2q33-q36 | RP11-803J6 | A549/CPT | 3(2) |
| | ABCB8 | | 7q35-q36 | RP11-606P1 | T24/cDDP10 | 5 (4) |
| | ABCB10 | | 1q42 | RP4-613A2 | HT29/CPT | 5(3) |
| | ABCB11 | BSEP | 2q24 | RP11-704B8 | A549/CPT | 3(2) |
| | ABCC1 | MRP1 | 16p13.1 | RP11-516F7 | HT-29/ETP | 5 (3) |
| | | | | | K562/etop20 | 4 (3) |
| | | | | | K562/etop80 | 4 (3) |
| | ABCC4 | MRP4 | 13q32 | RP11-124D15 | KK47/cDDP10 | 3 (2) |
| | ABCC9 | SUR2 | 12p12.1 | RP11-729I10 | SK3NP16 | 5 (2) |
| | | | | | SKOV3/VP | 5 (2) |
| | ABCD3 | PXMP1 | 1p21-p22 | RP11-272P3 | SKOV3/VP | 5 (3) |
| | ABCD4 | PXMPL1L | 14q24.3 | RP5-919J22 | HT-29/ADM | 3 (2) |
| | | | | | KK47/cDDP10 | 4 (3) |
| | | | | | K562/etop80 | 3 (2) |
| | ABCE1 | OABP | 4q31 | RP11-543H9 | HT-29/CPT | 4 (3) |
| | ABCF2 | | 7q35-q36 | RP4-548D19 | T24/cDDP10 | 5 (4) |
| Bc1-2 | | | | | | |
| | BCL2L2 | BCL-W | 14q11.2-q12 | RP11-124D2 | SKOV3/VP | 6 (2) |
| | MCL1 | | 1q21 | RP11-243G22 | HT-29/CPT | 4 (3) |
| | BCL2L10 | | 15q21 | RP11-337B11 | K562/AC | 4 (3) |

| | | | | | | |
|---|---|---|---|---|---|---|
| * copy number of resistant lines (copy number of parent lines thereof) | | | | | | |

In particular, in terms of copy number, amplification of 2 or more copies was observed in the ABCA3 gene, the ABCB1 (MDR1) gene, and the ABCC9 (SUR2) gene. In etoposide-resistant colon cancer HT 29/ETP cells, in which gene amplification had been detected in the chromosomal 16p12-p13 region by the CGH method, the copy number of the ABCA3 gene localizing in the chromosomal 16p13.3 region was calculated to be 7 copies by the FISH method, whereas the copy number of the ABCC1 (MRP1) gene localizing in the chromosomal 16p13.1 region was 5 copies. On the other hand, in the parent lines (HT-29) of HT-29/ETP cells, the copy number of both genes was 3 copies, which was a lower value than those as described above (Figures 1A and B). These results show that when compared with the resistant lines, the parent lines have higher sensitivity to etoposide. Applying the FISH method, the minimum chromosomal region, in which the ABCA3 gene amplified in etoposide-resistant colon cancer (HT-29/ETP) cells localized, was identified between BAC clone Nos. RP11-334D3 (including marker D16S525) and RP11-95P2 (including marker SHGC-11838) (Figure 1I). The copy number of the ABCB1 gene localizing in the chromosomal 7q21 region in etoposide-resistant lung cancer (SK3/VP16) cells was calculated to be 11 copies by the FISH method. This was a value significantly higher than that of the parent cells (3 copies). The copy number of the ABCC9 gene localizing the chromosomal 12p12.1 region was calculated to be 5 copies by the FISH method in both etoposide-resistant lung cancer (SK3/VP16 cells) and etoposide-resistant ovarian cancer (SKOV3/VP) cells. This was a value significantly higher than that of the parent lines (2 copies). It was considered that amplification of these genes induces a high level of expression of the mRNA thereof. Thus, it was then confirmed. RNA was extracted from parent lines and drug-resistant cells obtained from the parent lines. Thereafter, Northern blotting was performed thereon. As a result, it was found that expression of the ABCA3 gene was increased by 10.6 times and expression of the ABCC1 gene was increased by 2.6 times in etoposide-resistant HT-29/ETP cells, when compared with parent line colon cancer HT-29 cells (Figure 2A). From these results, it can be said that although both genes exist in the chromosomal 16p12-13 region, the ABCA3 gene is a more important gene than the ABCC1 gene, in that the ABCA3 gene imparts greater etoposide resistance than the ABCC1 gene does. Expression of the ABCB1 gene was 3.3 times higher in etoposide-resistant lung cancer (SK3/VP16) cells than in the parent cells (SK3) thereof. Expression of the ABCC9 gene was 13.3 times higher in SK3/VP16 cells than in the parent cells thereof. On the other hand, expression of the above gene was only 1.6 times higher in etoposide-resistant ovarian cancer (SKOV3/VP) cells than in the parent cells (SKOV3) thereof (Figure 2C). Accordingly, it is suggested that the type of an ABC transporter gene associated with drug resistance is different depending on the type of cells. In order to analyze drug resistance, it is necessary that a broad range of ABC transporter genes be comprehensively analyzed.

### [Example 3] Amplification of gene encoding anti-apoptotic protein belonging to BCL2 family and high expression thereof

A gene group consisting of the BCL2 gene, the BCL2L1 (BCLXL) gene, the BCL2L2 (BCLW) gene, the BCL2A1 gene, the MCL1 gene, and the BCL2L10 gene, belongs to a BCL2 family. These are genes encoding regulatory proteins having anti-apoptotic action (Gross, A., McDonnell, J. M., and Korsmeyers, S. J.: BCL-2 family members and the mitochondria in apoptosis, Genes Dev. 13, 1899-1911, 1999).

Gene amplification of 5 types of genes excluding the BCL2L1 (BCLXL) was observed in the drug resistant cells. In the analysis using FISH, when compared with each parent lines, amplification of the BCL2L2 gene took place in etoposide-resistant ovarian cancer (SKOV3NP) cells, amplification of the MCL1 gene took place in camtothecin-resistant colon cancer (HT-29/CPT) cells, and amplification of the BCL2L10 gene took place in cytosine arabinoside-resistant leukemia (K562/AC) cells (Table 2). In this case, the chromosomal 14q11-q21 region was amplified in etoposide-resistant ovarian cancer (SKOV3/VP) cells when compared with the patent cells thereof. The copy number of the BCL1L2 gene localizing in the chromosomal 14q11.2-q12 regions was calculated to be 6 copies by the FISH method (Figures 1C and 1D).

A hepatocyte nuclear factor 3 family gene, HNF3A gene, was mapped on the chromosomal 14q12 region, and it was found on the chromosomal 14q12-q13 region of esophageal cancer, esophageal squamous cell carcinoma, as an amplicon for inducing amplification of the above region. The copy number of the HNF3A gene in etoposide-resistant ovarian cancer cells was calculated to be 4 copies by the FISH method (Figure 1D). The BCL2L2 gene exists in a region that is highly likely to be an amplicon in the chromosomal 14q11-q21 region of etoposide-resistant cell line SKOV3/VP cells. Moreover, an amplicon comprising the BCL2L2 gene exists between the markers D14S879 (BAC clone RP11-146E13) and SHGC-10164 (BAC clone RP11-144C18) (Figure 1J). Expression of the BCL2L2 gene was analyzed by the Northern blot method. As a result, it was found that expression of the above gene was 3.3 times higher in SKOV3/VP cells than in the parent lines thereof (Figure 2B).

Subsequently, the gene expression of the aforementioned 6 types of BCL2 family genes were compared with one another in 22 types of drug-resistant cells (Figure 3). The BCL2 gene was expressed in 4 types of drug-resistant cells at 2 or more times higher level than in the parent lines thereof. Likewise, the BCL2L1 (BCLXL) gene was expressed in one type of drug-resistant cells, the BCL2L2 gene was expressed in 3 types of drug-resistant cancer cells, the MCL1 gene was expressed in one type of drug-resistant cancer cells, the BCL2A1 gene was expressed in one type of drug-resistant cancer cells, and the BCL2L10 gene was expressed in 5 types of drug-resistant cancer cells, at 2 or more times higher level than in each of their parent lines thereof (Figures 2B and 3).

The MCL1 gene was highly expressed in camptothecin-resistant colon cancer HT-29/CPT cells, and the BCL2L10 gene was highly expressed in cytosine arabinoside-resistant leukemia K562/AC cells, in proportional to gene amplification (Table 2 and Figure 3). Accordingly, it was revealed that high expression of the BCL2 family genes induces resistance to anticancer drugs, and that the analysis of the gene amplification is important to examine drug-resistant ability.

### [Example 4] Influence of BCL2L2 on apoptosis induced by etoposide VP-16)

The influence of BCL2L2 gene expression on apoptosis induced by anticancer drugs was analyzed. Human ovarian cancer SKOV3 cells and etoposide-resistant ovarian cancer SKOV3/VP cells were cultured for 24 hours in the presence of 100 µg/ml VP-16, so as to measure apoptosis associated with activation of caspase-3. As a result, the caspase activity was decreased to approximately one-half in etoposide-resistant SKOV3/VP cells than in the parent lines thereof (Figure 4A). Thereafter, in order to analyze the role of the BCL2L2 gene, the cells were treated with the antisense oligonucleotide of BCL2L2, and the influence of VP-16 was analyzed under conditions wherein expression of the BCL2L2 gene was suppressed. In the case of addition of only a transfection reagent (oligofectamine) or addition of a BCL2L2 sense oligonucleotide (a control oligonucleotide of a scramble sequence), there was no influence. However, when a BCL2L2 antisense oligonucleotide was added, expression of the BCL2L2 gene was decreased (Figure 4B). Under such conditions, the sensitivity of the SKOV3/VP cells to etoposide (VP-16) was increased (Figure 4C). Accordingly, it can be said that amplification of the BCL2 family genes reduces caspase activity, and thud resistance to anticancer drugs is induced.

### [Example 5] Gene copy numbers of DCK, TOP1 and TOP2A, and expression levels thereof

Cytosine arabinoside-resistant leukemia K562/AC cells comprise a deletion of the chromosomal 4q region where the deoxycytidine (DCK) gene is localized. Camptothecin-resistant colon cancer HT-29/CPT cells comprise a deletion of the chromosomal 20q region wherein topoisomerase 1 (TOP1) is localized. On the other hand, neither amplification nor deletion of the chromosomal 17q21-22 region wherein TOP2A is localized is observed in 5 types of etoposide (VP-16)-resistant cells (Table 1). The copy numbers of the DCK gene, TOP1 gene and TOP2A gene were obtained by the FISH method in cells resistant to cytosine arabinoside (Ara-C), camptothecin (CPT), and etoposide (VP-16). The expression levels thereof were then measured by the real-time reverse transcription PCR method (Table 3).

**(Table 3) Copy numbers of DCK gene in cytosine arabinoside-resistant cell line, TOP1 gene in camptothecin-resistant cell line, and TOP2A gene in etoposide-resistant cell line, and gene expression levels thereof**

| Gene name | Localized chromosomal region | BAC/PAC name | Cell line | DNA copy number* | Relative gene expression level** |
|---|---|---|---|---|---|
| DCK | 14q13-21 | RP11-499N1 | K562/AC | 1(2) | 0.00 |
| TOP1 | 20q12-13 | RP1-1J6 | HT-29/CPT | 2 (5) | 0.36 |
| | | | A549/CPT | 3 (3) | 0.47 |
| | | | St-4/CPT | 3(6) | 0.54 |
| TOP2A | 17q21-22 | RP11-58O9 | HT-29/ETP | 2 (3) | 0.37 |
| | | | K562/etop20 | 2 (3) | 0.48 |
| | | | K562/etop80 | 2 (3) | 0.69 |
| | | | SK3/VP16 | 4 (3) | 0.44 |
| | | | SKOV3/VP | 5 (4) | 0.61 |

| | | | | | |
|---|---|---|---|---|---|
| * DNA copy number is expressed in the form of a copy number in resistant lines (copy number in parent cell lines). ** The ratio between the expression level of the target gene and the expression level of a control gene (GAPDH) is obtained. The value of the parent cell line of each drug-resistant cell is defined as 1, and the relative gene expression level is expressed in the form of a ratio to the above value. (Abbreviated symbols) DCK: Deoxycytidine kinase gene TOP1: Topoisomerase 1 gene TOP2A: Topoisomerase 2A gene | | | | | |

The copy number of the DCK gene in cytosine arabinoside-resistant leukemia (K562/AC) cells was calculated to be 1 copy by the FISH method, when compared with in the parent lines thereof. This shows that a pair of the genes out of two pairs of the genes in Ara-C-resistant cells were deleted (Figures 1E and 1F). Expression of the DCK gene was clearly detected in human leukemia K562 cells, but it was not detected in cytosine arabinoside-resistant leukemia K562/AC cells. Colon cancer HT-29 cells have 5 copies of TOP1 genes, whereas camptothecin-resistant colon cancer HT-29/CPT cells have 2 copies of the above genes. Thus, the copy number in the camptothecin-resistant colon cancer HT-29/CPT cells was significantly decreased (Figures 1G and 1H). Likewise, the copy number of TOP1 was 6 copies in parent line stomach cancer (St-4) cells, but the above copy number was decreased to 3 copies in camptothecin-resistant stomach cancer (St-4/CPT) cells. In all 3 types of camptothecin-resistant cell lines, the copy number was decreased to 3 copies or less. The copy number of the TOP2A gene was lower in camptothecin-resistant colon cancer HT-29/ETP cells and in etoposide-resistant leukemia (K562/etop20 and K562/etop80) cells than in their parent lines thereof. Expression of the TOP2A gene was decreased in all 5 types of etoposide-resistant cell lines.

Thus, a decrease in the copy numbers of the DCK gene, TOP1 gene, and TOP2A gene is closely associated with resistance to anticancer drugs. The analysis of amplification and deletion of these genes on the chromosome is extremely important to examine the anticancer drug-resistant ability of cancer cells.

### [Example 6] Production of CGH array for diagnosing drug-resistant ability of cancer cells and cancer tissues

ABCA3 gene, ABCB1 gene, ABCB6 gene, ABCB8 gene, ABCB10 gene, ABCB11 gene, ABCC1 gene, ABCC4 gene, ABCC9 gene, ABCD3 gene, ABCD4 gene, ABCE1 gene, and ABCF2 gene, all of which belong to the ABC transporter family, are contained in BAC clones, namely, RP11-304L19, RP11-212B1, RP11-803J6, RP11-606P1, RP4-613A2, RP11-704B8, RP11-516F7, RP11-124D15, RP11-729I10, RP11-272P3, RP5-919J22, RP11-543H9, and RP4-548D19, respectively. BCL2L2 gene, MCL1 gene, and BCL2L10 gene, which belong to the BCL2 family, are contained in BAC clones, namely, RP11-124D2, RP11-243G22, and RP11-337B11, respectively. The BAC DNA of each of these genes was digested with 4 nucleotides recognition enzyme RsaI, and an adapter was then added thereto, so as to conduct ligation. Thereafter, PCR was carried out using adapter sequences as primers, so as to carry out gene amplification (this amplification process is referred to as a "process of inexhaustibly producing gene amplified products"). As control DNA, 5 types of BAC DNA (RP11-98A20, RP11-252011, RP11-357G3, RP11-196F4, and RP11-736A8) other than the aforementioned BAC DNA were inexhaustibly produced.

The thus inexhaustibly produced DNA was spotted on a slide glass used for an amination oligonucleotide-fixing array in a triplicate manner, using an ink-jet printer, so as to produce a DNA-fixed substrate.

DNA derived from the tissues of a healthy subject is labeled with Cy5, whereas DNA derived from cancer tissues excised from a cancer patient is labeled with Cy3. The mixed solution thereof is subjected to hybridization on a DNA-fixed substrate, and the array is then scanned. Thereafter, the Cy3 signal and the Cy5 signal are standardized, and the intensity ratio of Cy3/Cy5 is then obtained. When the ratio is 2, it indicates that the genomic region including genes printed on the DNA-fixed substrate is amplified by 2 times in cancer tissues. When the ratio is 4, it indicates that the above genomic region is amplified by 4 times in cancer tissues.

### INDUSTRIAL APPLICABILITY

Human ABC transporter genes, BCL2 family genes, and DNA synthesis-associated genes, are closely associated with anticancer drug resistance. Thus, according to the present invention, amplification and deletion of these gene regions are examined on the chromosome of a cancer cell, so that the resistance of the cancer cell to anticancer drugs can be determined. As a result, it becomes possible to easily diagnose whether or not the cell on the affected area of a cancer patient is resistant to anticancer drugs. This enables chemotherapy suitable for individual patients, that is, made-to-order medicine. This is able to avoid chemotherapy with extremely low efficiency based on acquisition of anticancer drug-resistant ability, and is also able to prevent a useless decrease in the quality of life of patients.

## Claims

1. A detection method of detecting acquisition of the drug resistance of a test cancer cell to anticancer drugs, which comprises detecting amplification of one or more types of genes selected from ABC transporter genes and BCL2 family genes consisting of ABCA3 gene, ABCB6 gene, ABCB8 gene, ABCB10 gene, ABCC4 gene, ABCC9 gene, ABCD3 gene, ABCD4 gene, ABCE1 gene, ABCF2 gene, BCL2L2, BCL2L10, BCL2L1, and BCL2A1, in said test cancer cell.

2. The detection method according to claim 1, wherein amplification of the ABCA3 gene is an index of acquisition of drug resistance to etoposides, amplification of the ABCB6 gene is an index of acquisition of drug resistance to camptothecins, amplification of the ABCB8 gene is an index of acquisition of drug resistance to cisplatins, amplification of the ABCB10 gene is an index of acquisition of drug resistance to camptothecins, amplification of the ABCC4 gene is an index of acquisition of drug resistance to cisplatins, amplification of the ABCC9 gene is an index of acquisition of drug resistance to etoposides, amplification of the ABCD3 gene is an index of acquisition of drug resistance to etoposides, amplification of the ABCD4 gene is an index of acquisition of drug resistance to adriamycins, amplification of the ABCE1 gene is an index of acquisition of drug resistance to camptothecins, amplification of the ABCF2 gene is an index of acquisition of drug resistance to cisplatins, amplification of the BCL2L2 gene is an index of acquisition of drug resistance to camptothecins, cisplatins, etoposides, or cytosine arabinosides, amplification of the BCL2L10 gene is an index of acquisition of drug resistance to camptothecins, cisplatins, or cytosine arabinosides, and amplification of the BCL2L1 gene is an index of acquisition of drug resistance to camptothecins, cisplatins, etoposides, or cytosine arabinosides.

3. The detection method according to claim 1 or 2, wherein detection is carried out by the CGH method, the flow cytometry method, the ELISA method, the DNA chip method, or the quantitative PCR method.

4. The detection method according to claim 1 or 2, wherein detection is carried out by the CGH method or the DNA chip method.

5. The detection method according to claim 4, wherein a substrate used in the CGH method or the DNA chip method is a DNA fixed substrate wherein the DNA comprises one or more types of genes selected from ABC transporter genes and BCL2 family genes consisting of ABCA3 gene, ABCB6 gene, ABCB8 gene, ABCB10 gene, ABCC4 gene, ABCC9 gene, ABCD3 gene, ABCD4 gene, ABCE1 gene, ABCF2 gene, BCL2L2 gene, BCL2L10 gene, BCL2L1 gene, and BCL2A1 gene.

6. The detection method according to claim 5, wherein said substrate is a DNA fixed substrate which further comprises one or more types of genes selected from ABC transporter genes, BCL2 family genes, and DNA synthesis-associated genes, which consist of ABCB1 gene, ABCC1 gene, ABCB11 gene, BCL2 gene, MCL1 gene, BCLXL gene, DCK1 gene, TOP1 gene, and TOP2A gene.

7. The method of detecting drug resistance-acquired cancer cells according to any one of claims 4 to 6, which comprises: allowing control DNAs and the DNA of a test cancer cell used as a target of detection of acquisition of drug resistance, each of which was labeled with each different fluorescent dye, to simultaneously contact with said DNA-fixed substrate, so as to conduct hybridization; and quantitatively detecting amplification or deletion of a specific region of the test DNA by using the fluorescent dye obtained as a result of the hybridization as an index.

8. The method of detecting drug resistance-acquired cancer cells according to claim 7, wherein DNA fixed on said DNA-fixed substrate, test DNA, and control DNA are genomic DNAs.

9. A DNA-fixed substrate on which DNA comprising one or more types of genes selected from ABC transporter genes and BCL2 family genes consisting of ABCA3 gene, ABCB6 gene, ABCB8 gene, ABCB10 gene, ABCC4 gene, ABCC9 gene, ABCD3 gene, ABCD4 gene, ABCE1 gene, ABCF2 gene, BCL2L2 gene, BCL2L10 gene, BCL2L1 gene, and BCL2A1 gene, is fixed.

10. The DNA-fixed substrate according to claim 8, on which several types of DNAs comprising one or more types of genes selected from ABC transporter genes, BCL2 family genes, and DNA synthesis-associated genes, which consist of ABCB1 gene, ABCC1 gene, ABCB11 gene, BCL2 gene, MCL1 gene, BCLXL gene, DCK1 gene, TOP1 gene, and TOP2A gene, are further fixed.

11. The DNA-fixed substrate according to claim 9 or 10, wherein several types of DNAs that are to be fixed on the substrate are genomic DNAs, cDNAs, or synthetic oligonucleotides.

12. The DNA-fixed substrate according to claim 11, wherein several types of DNAs are genomic DNAs and wherein said genomic DNAs are gene amplified products of BAC DNA, YAC DNA, or PAC DNA.
